# EUROPEAN PATENT APPLICATION

(11) **EP 4 729 037 A1**
(43) Date of publication of application: **22.04.2026**
(21) Application number: 24845688.1
(22) Date of filing: 26.07.2024
(51) Int. Cl.: A61F 11/00

(54) **VESTIBULAR FUNCTION IMPROVEMENT DEVICE AND VESTIBULAR FUNCTION IMPROVEMENT METHOD**

(30) Priority: 27.07.2023 JP 2023122516
(71) Applicant: KOWA COMPANY, LTD., Aichi 460-8625 (JP)
(72) Inventor: YABUSAKI, Katsumi, Higashimurayama-shi Tokyo 189-0022 (JP); TAKEUCHI, Nobuhiko, Higashimurayama-shi Tokyo 189-0022 (JP)
(74) Representative: Kronthaler, Schmidt & Coll.
(86) International application number: PCT/JP2024/026739
(87) International publication number: WO 2025/023314

(57) **Abstract**

Vestibular function improvement device (100) of the present invention includes a generation unit (1) that generates an electric signal that causes a vibration including a frequency component in a range of 450 to 1500 hertz, and an output unit (2) that outputs a stimulus based on the electric signal. The stimulus output by the output unit (2) is transmitted to the vestibular system of the inner ear. According to the present invention, vestibular functions can be improved by stimulating the vestibular system of the inner ear.

## Description

### Technical Field

The present invention relates to a vestibular function improvement device and a vestibular function improvement method that improve the vestibular function by providing stimulus to the vestibular system of an inner ear.

### Background Art

The inner ear is composed of a vestibular system (otolith organs and semicircular canals) and a cochlea, and the vestibular system is an organ responsible for an equilibrium function of human. In particular, the otolith organs (utricle and saccule), which sense head tilt (gravitational acceleration) and linear acceleration, function to enable humans to maintain their sense of equilibrium. Numerous studies have revealed that any impairment of this equilibrium function can cause symptoms of dizziness. Furthermore, a decline in the equilibrium function of vestibular, or the vestibular function, can lead to conditions other than dizziness, such as locomotive syndrome, and can also lead to a susceptibility to car sickness, sea sickness, alcohol sickness, etc., which can occur even in healthy individuals.

It is known that applying sound stimuli to the vestibular system to reduce dizziness can improve the vestibular function. For example, Patent Document 1 describes a method of improving vertigo through generating a sound stimulus of a specific frequency (50 to 140 hertz) at a specific volume level (70 to 85 decibels) as a sound stimulus and stimulating the otoliths of a patient suffering from vertigo with the sound stimulus.

### Prior Art Documents

### Patent Documents

Patent Document 1: WO2019/230941

### Summary of the Invention

### Problems to be solved by the Invention

The present inventors have conducted extensive research into whether it is possible to provide a stimulus to the inner ear vestibular system that is more suitable for improving the vestibular function, and have completed the present invention. That is, an object of the present invention is to provide a vestibular function improvement device and a vestibular function improvement method that are able to improve the vestibular function by providing a stimulus to the inner ear vestibular system.

### Means for solving the Problems

First, the present invention provides a vestibular function improvement device comprising: a generation unit that generates an electric signal that causes a vibration including a frequency component in a range of 450 to 1500 hertz; and an output unit that outputs a stimulus based on the electric signal (Claim 1).

As a result of extensive research, the present inventors have found that the vestibular function can be improved by applying a stimulus including a frequency component in a range of 450 to 1500 hertz. That is, according to the above invention (Claim 1), the vestibular function can be improved through outputting a stimulus including a frequency component in a range of 450 to 1500 hertz, which is suitable for improving the vestibular function, and applying the stimulus to the vestibular system of the inner ear.

In the above invention (Claim 1), it is preferred that the stimulus output by the output unit should be transmitted to the vestibular system of an inner ear (Claim 2).

In the above invention or inventions (Claims 1 and 2), the stimulus may be a sound stimulus (Claim 3), or the stimulus may also be a vibration stimulus (Claim 4).

In the above invention or inventions (Claims 1 to 4), the output unit outputs the stimulus as noise including frequency components across a predetermined range (Claim 5).

In the above invention (Claim 5), it is preferred that the sound pressure level of a high-frequency band in the noise should be lower than the sound pressure level of a frequency band in a range of 450 to 1500 hertz (Claim 6). In the above invention (Claim 6), the high-frequency band may be a frequency band of 2000 hertz or higher (Claim 7).

In the above invention or inventions (Claims 5 to 7), the output unit may output the stimulus in a form of Brownian noise or pink noise (Claim 8).

In the above invention (Claim 1), the stimulus may be a vibration stimulus, and the output unit may output the stimulus in a form of white noise (Claim 9).

In the above invention or inventions (Claims 1 to 9), the output unit may output the stimulus after mixing it with a sound different from the stimulus (Claim 10).

Second, the present invention provides a vestibular function improvement method comprising: generating an electric signal that causes a vibration including a frequency component in a range of 450 to 1500 hertz; outputting a stimulus based on the electric signal; and transmitting the stimulus to a vestibular system of an inner ear (Claim 11).

### Advantageous Effect of the Invention

According to the vestibular function improvement device and vestibular function improvement method of the present invention, the vestibular function can be improved through outputting a stimulus including a frequency component in a range of 450 to 1500 hertz, which is suitable for improving the vestibular function, and applying the stimulus to the vestibular system of the inner ear. The improvement of the vestibular function leads to effects such as alleviating dizziness caused by diseases such as benign paroxysmal positional vertigo, Meniere's disease, and vestibular neuritis, as well as improving diseases and symptoms other than dizziness, for example, the symptoms of locomotive syndrome, and alleviating dizziness caused by car sickness, sea sickness, alcohol sickness, etc., which can occur even in healthy individuals.

### Brief Description of the Drawings

[FIG. 1] FIG. 1 is a schematic diagram of a vestibular function improvement device according to a first embodiment.
[FIG. 2] FIG. 2 is a diagram illustrating the configuration of a device main body of the vestibular function improvement device according to the same embodiment.
[FIG. 3] FIG. 3 is a functional block diagram illustrating functions that play a major role in the device main body of the vestibular function improvement device according to the same embodiment.
[FIG. 4] FIG. 4 is a schematic diagram of a vestibular function improvement device according to a second embodiment.
[FIG. 5] FIG. 5 is a set of diagrams for describing a scheme for evaluating the results of center of gravity sway measurement.
[FIG. 6] FIG. 6 is a graph illustrating the results of Experiment 1 to verify effectiveness.
[FIG. 7] FIG. 7 is a graph illustrating the results of Experiment 2 to verify effectiveness.
[FIG. 8] FIG. 8 is a set of graphs (part 1) illustrating the noise applied to the subject.
[FIG. 9] FIG. 9 is a set of graphs (part 2) illustrating the noise applied to the subject.
[FIG. 10] FIG. 10 is a set of graphs (part 3) illustrating the noise applied to the subject.
[FIG. 11] FIG. 11 is a graph illustrating the results of Experiment 12 to verify effectiveness.
[FIG. 12] FIG. 12 is a graph illustrating the results of Experiment 13 to verify effectiveness.

### Embodiments for Carrying out the Invention

Hereinafter, embodiments of the present invention will be described in detail with reference to the drawings. The embodiments described below are merely exemplified, and the present invention is not limited to these embodiments.

### First Embodiment

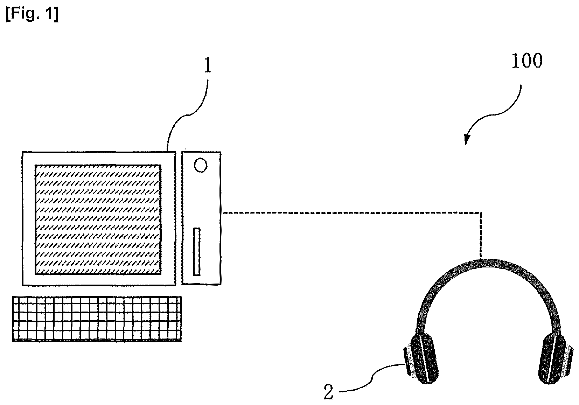

As illustrated in FIG. 1, vestibular function improvement device 100 according to the present embodiment includes a device main body 1 for generating an electrical signal that causes vibrations containing frequency components in a range of 450 to 1500 hertz, and headphones 2 for outputting a stimulus based on the electrical signal. In the vestibular function improvement device 100 of the present embodiment, a recipient seeking to improve the vestibular function wears the headphones 2, and the stimulus is transmitted in the form of a sound stimulus to the vestibular system of the recipient's inner ear.

The device main body 1 is configured to generate an electrical signal that causes vibrations containing frequency components in a range of 450 to 1500 hertz. The device main body 1 is an example of a generation unit in the present invention, and a general-purpose personal computer can be used, for example, but the device main body 1 is not limited to this. It may also be a mobile information terminal such as a smartphone or tablet, or a dedicated terminal device.

The electrical signal generated by the device main body 1 may be a signal that generates a vibration corresponding to a pure tone having a specific frequency component in a range of 450 to 1500 hertz, or a signal that generates vibrations corresponding to a complex tone including specific frequency components at least in a range of 450 to 1500 hertz. The complex tone including specific frequency components at least in a range of 450 to 1500 hertz may be a combination of multiple pure tones, or may be noise including frequency components across a predetermined range.

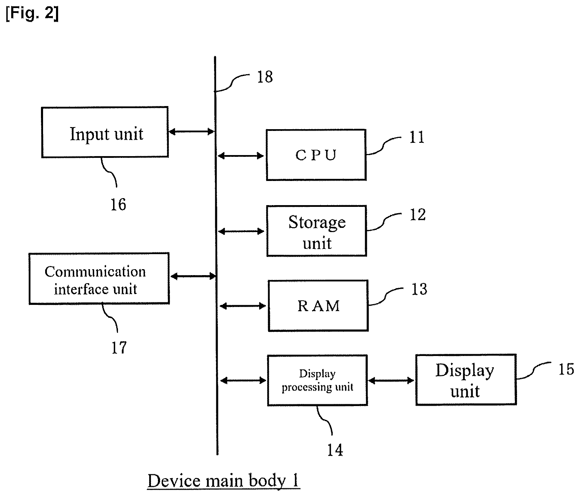

As illustrated in FIG. 2, the device main body 1 includes a CPU 11, a storage unit 12, a RAM 13, a display processing unit 14, a display unit 15, an input unit 16, and a communication interface unit 17, and a bus 18 is also provided for transmitting control signals or data signals between these units.

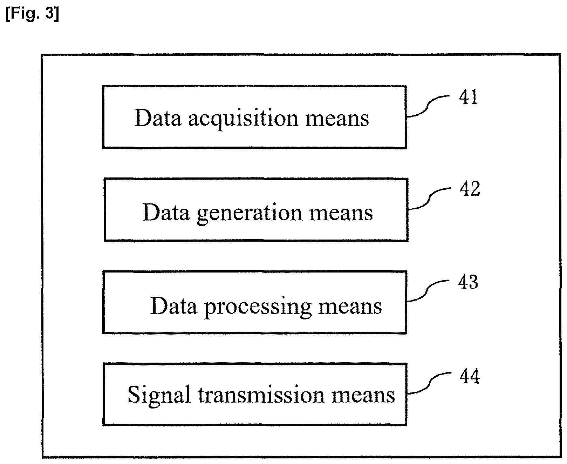

When power is applied to the device main body 1, the CPU 11 loads various programs stored in the storage unit 12 into the RAM 13 and executes them. In the present embodiment, the CPU 11 reads and executes the programs stored in the storage unit 12, thereby achieving the functions of a data acquisition means 41, a data generation means 42, a data processing means 43, and a signal transmission means 44 (illustrated in FIG. 3), which will be described later.

The display processing unit 14 displays data for display provided by the CPU 11 on the display unit 15. The display unit 15 for use can be, for example, an LCD display.

The input unit 16 has a set of buttons for receiving user input operations and includes an interface circuit for recognizing inputs of button presses (operations) and outputting them to the CPU 11, but a touch panel input scheme may also be employed for the input unit 16.

The communication interface unit 17 is connected to an external device (not illustrated) that supplies sound data to the device main body 1, a sound-collecting microphone (not illustrated) that acquires sound data, and other devices, and the communication interface unit 17 is configured to allow the device main body 1 to acquire sound data from outside. The headphones 2 are connected to the communication interface unit 17, which is configured to be able to transmit an electrical signal that generates vibrations to the headphones 2.

The functions achieved by the device main body 1 configured as described above will now be described. FIG. 3 is a functional block diagram illustrating functions that play a major role in the device main body 1 of the present embodiment, and includes the data acquisition means 41, the data generation means 42, the data processing means 43, and the signal transmission means 44. Not all of these means are essential components of the device main body 1, and the functions of the device main body 1 can be modified as appropriate, such as by allowing the device main body 1 to be selectively equipped with any of these functions.

The data acquisition means 41 has a function of acquiring sound data from outside the device main body 1, and this function is achieved, for example, as follows. When a data import instruction is input via the input unit 16, the CPU 11 of the device main body 1 imports sound data from an external device (not illustrated) or a sound-collecting microphone (not illustrated) connected to the communication interface unit 17, and stores the imported sound data in the storage unit 12. The sound data may be noise, music, human or animal voices, environmental sounds, artificial sounds, or other sounds recorded in a digital file in a predetermined format, or may also be data imported into the device main body 1 via a sound-collecting microphone or the like.

The data generation means 42 has a function of mechanically generating sound data in the device main body 1, and this function is achieved, for example, as follows. When a data generation instruction is input via the input unit 16, the CPU 11 of the device main body 1 operates to mechanically generate sound data of a pure tone having a specific frequency component or sound data of noise having a specific waveform, and stores the generated sound data of the pure tone or noise in the storage unit 12.

The data processing means 43 has a function of processing the sound data stored in the storage unit 12, and this function is achieved, for example, as follows. When an instruction to process sound data is input via the input unit 16, the CPU 11 of the device main unit 1 selects the sound data to be processed from the sound data stored in the storage unit 12, performs the processing specified for the sound data, and stores the processed sound data back in the storage unit 12. The content of processing may be, for example, processing of mixing multiple sound data, processing of cutting a certain frequency band of the sound data, processing of attenuating a certain frequency band of the sound data, or processing of emphasizing a certain frequency band of the sound data. When issuing an instruction for each processing operation, the waveform of the selected sound data may be displayed on the display unit 15, allowing the user to operate the input unit 16 while confirming the display unit 15.

The signal transmission means 44 has a function of converting the sound data into an electrical signal and transmitting it to the outside of the device main body 1, and this function is achieved, for example, as follows. When an instruction to convert the sound data into an electrical signal and transmit it to the outside is input via the input unit 16, the CPU 11 of the device main unit 1 selects the sound data to be transmitted from the sound data (both unprocessed and processed) stored in the storage unit 12, converts the selected sound data into an electrical signal, and transmits it from the communication interface unit 17 to the outside of the device main unit 1.

The headphones 2 are electrically connected to the device main unit 1 via a wired or wireless connection, and are configured to receive the electrical signal generated in the device main unit 1 and output a stimulus based on the electrical signal. The headphones 2 are an example of the output unit in the present invention, and may be earphones, speakers, or other audio devices. The speakers may be stationary or portable, such as worn around the neck, or may be built into hearing aids, glasses, sunglasses, a chair, a pillow, an artificial inner ear, etc.

In the present embodiment, the device main body 1 (generation unit for an electrical signal) and headphones 2 (output unit for a stimulus) constituting the vestibular function improvement device 100 are physically separate, but they can also be implemented by integrating them into a single device. For example, the vestibular function improvement device may be implemented using a smart device such as a smart speaker, may be developed as a dedicated vestibular function improvement device with a built-in speaker as the output unit, or may be implemented as a hearing aid with a built-in mechanism for playing a therapeutic stimulus. When the vestibular function improvement device 100 is implemented as a hearing aid, the hearing aid picks up ambient sounds, processes them to make them easier to hear, and transmits them to the wearer via earphones, but the hearing aid may be configured to preliminarily prepare a sound stimulus to be synthesized with the sounds in the hearing aid or supply it from outside and provide the wearer with the sound stimulus along with the ambient sounds.

The sound stimulus output by the headphones 2 is sound containing frequency components in a range of 450 to 1500 hertz. This sound stimulus may be a pure tone containing a specific frequency component in a range of 450 to 1500 hertz, or may also be a complex tone containing at least specific frequency components in a range of 450 to 1500 hertz. The complex tone containing at least specific frequency components in a range of 450 to 1500 hertz may be a composite of multiple pure tones, or may also be noise containing frequency components across a predetermined range. By providing the recipient with a sound stimulus containing frequency components in a range of 450 to 1500 hertz, the recipient's vestibular function can be improved.

When the sound stimulus is noise containing frequency components across a predetermined range, it is preferred that the sound pressure level of the high-frequency band in the noise should be lower than the sound pressure level of the frequency band in a range of 450 to 1500 hertz. In particular, it is preferred that the sound pressure level of a high-frequency band of 2000 hertz or higher should be lower than the sound pressure level of the frequency band in a range of 450 to 1500 hertz. Rather than providing the recipient with noise containing high-frequency components as a sound stimulus, providing the recipient with noise, in which high-frequency components are removed or attenuated, as a sound stimulus is more effective in improving the recipient's vestibular function.

There are various types of noise, including those found in nature and those generated mechanically. In particular, noise that exhibits a constant power spectral density or has a feature of attenuation or amplification depending on the frequency is referred to by various color names and is generally called colored noise (chromatic noise). Noise that has the same intensity (power spectral density) across all frequency bands is called white noise. In contrast to the white noise, noise that has a feature of attenuation in the high-frequency band is called pink noise (power spectral density inversely proportional to frequency) or Brownian noise (power spectral density inversely proportional to the square of frequency). On the other hand, noise that has a feature of amplification in the high-frequency band is called blue noise (power spectral density is proportional to frequency) or purple noise (power spectral density is proportional to the square of frequency).

Noises that have a strong effect on improving the vestibular function include those in which the sound pressure level of the high-frequency band is lower than that of a frequency band in a range of 450 to 1500 hertz. In particular, Brownian noise is more pleasant to listen to than pure tone, thus reducing stress experienced by the recipient during treatment. Furthermore, even white noise processed to cut out high-frequency bands has been confirmed to have a significant effect on improving the vestibular function.

The frequency at which attenuation begins may be set arbitrarily. For example, the noise may have a constant power spectral density below 100 hertz and attenuate at higher frequencies. Setting the frequency at which attenuation begins can be expected to enhance comfort.

Furthermore, the noise is not limited to Brownian noise or pink noise, and it may be noise with a decimal exponent, such as power spectral density inversely proportional to the 1.5th power of frequency. Specifically, noise with an exponent of 0.5 or 2.5 is conceivable.

Alternatively, other than the noise that attenuates according to a frequency rule as described above, noise may be that in which one or more frequency bands of an arbitrary width are attenuated or band-cut processed, or that which attenuates in a step-like manner with each step width set to an arbitrary value for each predetermined frequency. For example, noise may be that which has frequency characteristics generated to draw a free-form curve or the like that follows the frequency characteristics of the human audible range.

Providing a pure tone or noise alone as the sound stimulus may be distressing for some recipients. For this reason, processing may be performed to mix pleasant sounds, for example, music or environmental sounds, with sounds containing frequency components in a range of 450 to 1500 hertz, and the processed sound stimulus may be provided to the recipient. Such mixed sound stimulus allows the recipient to receive the sound stimulus more comfortably.

The sound pressure level of the sound stimulus output by the headphones 2 is not particularly limited, but is preferably in a range of 10 to 70 decibels and more preferably in a range of 20 to 50 decibels. A sound pressure level below 10 decibels will not provide a sufficient therapeutic effect, while a sound pressure level above 70 decibels will not only be painful for the recipient, but may also cause hearing loss in some cases.

The duration for which the recipient is stimulated by the sound stimulus output by the headphones 2 is not particularly limited, but the stimulus is preferably maintained for a period of 30 seconds to 60 minutes and more preferably for a period of 1 to 10 minutes. If the stimulus duration is less than 30 seconds, the duration of the therapeutic effect (carryover effect) will be short, and sufficient therapeutic effect cannot be obtained. On the other hand, if the stimulus duration is longer than 60 minutes, although a therapeutic effect will be obtained, no significant increase in the carryover effect is observed, and further extension of the duration is not expected to be effective. Furthermore, the length of time spent restrained may increase the burden on the patient.

The description will now be directed to a procedure for using the vestibular function improvement device 100 configured as described above to treat a recipient (such as a vertigo patient) to improve the vestibular function. First, the treating person (e.g., a caregiver such as a physician) powers on the device main unit 1 of the vestibular function improvement device 100 and has the recipient wear the headphones 2. Subsequently, the treating person operates the device main unit 1 to output from the headphones 2 a sound stimulus containing frequency components in a range of 450 to 1500 hertz appropriate for the recipient. The recipient listens to the output sound thereby to allow the recipient's vestibular function to be activated, and for example, if the recipient is a vertigo patient, the patient's dizziness is alleviated. The sound stimulus may be output continuously for a predetermined period of time, or may be output intermittently a plurality of times with intervals in between. The conditions for presenting the sound stimulus can be determined appropriately based on the recipient's illness, symptoms, severity of symptoms, purpose of providing the stimulus, etc. The recipient him/herself may also operate the vestibular function improvement device 100 as the treating person.

### Second Embodiment

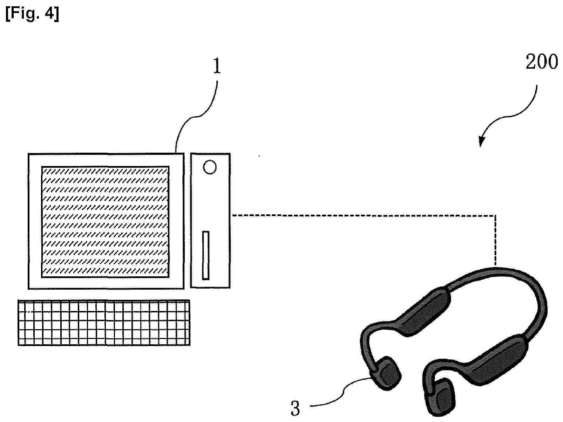

As illustrated in FIG. 4, vestibular function improvement device 200 according to the present embodiment includes a device main body 1 for generating an electrical signal that causes vibrations containing frequency components in a range of 450 to 1500 hertz, and bone conduction earphones 3 for outputting a stimulus based on the electrical signal. In the vestibular function improvement device 200 of the present embodiment, a recipient wears the bone conduction earphones 3, and the stimulus is transmitted in the form of vibration stimulus to the vestibular system of the recipient's inner ear. The device main unit 1 is similar to that of the vestibular function improvement device 100 of the first embodiment, and therefore its description is omitted here.

The bone conduction earphones 3 are electrically connected to the device main unit 1 via wired or wireless connections, and are configured to receive the electrical signal generated by the device main unit 1 and output a stimulus based on the electrical signal. The bone conduction earphones 3 are an example of the output unit in the present invention, and transmit vibrations directly to the inner ear by applying vibrations to the ear and surrounding sites. The output unit used to transmit the vibration stimulus may also be a device that applies vibrations by contacting any site of the head other than the ear of the recipient, for example, the mandible, nasal bone, mastoid process, teeth, etc., in which case, devices shaped like glasses, hairbands, or headgear may be used. Alternatively, the device may incorporate a vibrator. The vibrator may be built into a hearing aid, glasses, sunglasses, chair, pillow, artificial inner ear, or the like. If the vibration stimulus can be applied to the recipient without blocking the ear, the vestibular function can be improved while the recipient is able to hear external sounds.

The vibration stimulus output by the bone conduction earphones 3 is a vibration of sound containing frequency components in a range of 450 to 1500 hertz. This vibration stimulus may be a vibration of pure tone containing a specific frequency component in a range of 450 to 1500 hertz, or may also be vibrations of complex tone containing at least specific frequency components in a range of 450 to 1500 hertz. The vibrations of complex tone containing at least specific frequency components in a range of 450 to 1500 hertz may be a composite of vibrations of multiple pure tones, or may also be vibrations of noise containing frequency components across a predetermined range. By providing the recipient with a vibration stimulus containing frequency components in a range of 450 to 1500 hertz, the recipient's vestibular function can be improved.

When the vibration stimulus is a vibration of noise containing frequency components across a predetermined range, it is preferred that the sound pressure level of the high-frequency band in the noise should be lower than the sound pressure level of the frequency band in a range of 450 to 1500 hertz. In particular, it is preferred that the sound pressure level of a high-frequency band of 2000 hertz or higher should be lower than the sound pressure level of the frequency band in a range of 450 to 1500 hertz. Rather than providing the recipient with noise containing high-frequency components as a vibration stimulus, providing the recipient with noise, in which high-frequency components are removed or attenuated, as a vibration stimulus is more effective in improving the recipient's vestibular function.

Noises that have a strong effect on improving the vestibular function include those in which the sound pressure level of the high-frequency band is lower than that of a frequency band in a range of 450 to 1500 hertz. Furthermore, even white noise processed to cut out high-frequency bands has been found to have a significant effect on improving the vestibular function.

Furthermore, when applying the vibration stimulus to the recipient, unlike the sound stimulus, even white noise that is not processed to cut out high-frequency bands has been confirmed to have a certain degree of effect on improving the vestibular function. This is thought to be because the bone conduction propagation path is complex, and high-frequency bands are attenuated by skin, bone tissue, etc. in the process of the vibrations reaching the inner ear, resulting in that the vibration stimulus is transmitted to the inner ear as noise with frequency characteristics similar to those of Brownian noise.

The description will now be directed to a procedure for using the vestibular function improvement device 200 configured as described above to treat a recipient (such as a vertigo patient) to improve the vestibular function. First, the treating person (a caregiver such as a physician, etc.) powers on the device main unit 1 of the vestibular function improvement device 200 and has the recipient wear the bone conduction earphones 3. Subsequently, the treating person operates the device main unit 1 to output from the bone conduction earphones 3 a vibration stimulus obtained by converting to a vibration a sound containing frequency components in a range of 450 to 1500 hertz appropriate for the recipient. The output vibration is transmitted to the recipient's vestibular system thereby to activate the recipient's vestibular function, and for example, if the recipient is a vertigo patient, the patient's dizziness is alleviated. The vibration stimulus may be output continuously for a predetermined period of time, or may be output intermittently a plurality of times with intervals in between. The conditions for presenting the vibration stimulus can be determined appropriately based on the recipient's illness, symptoms, severity of symptoms, purpose of providing the stimulus, etc. The recipient him/herself may also operate the vestibular function improvement device 200 as the treating person.

The vestibular function improvement device and vestibular function improvement method according to the present invention have been described above based on the drawings, but the present invention is not limited to the above embodiments, and various modifications are possible. For example, the number of stimuli applied to the recipient does not need to be limited to one, and both the sound stimulus and the vibration stimulus may be applied simultaneously, or the sound stimulus may be applied first, followed by the vibration stimulus after a predetermined time.

### Various Experiments and Evaluation Results

Through various experiments, the inventors have verified and confirmed that outputting the aforementioned stimulus using the vestibular function improvement device 100, 200 and applying the stimulus to the inner ear vestibular system effectively improves the vestibular function. The following description is directed to the various experiments conducted by the inventors and their evaluation results.

### Evaluation Method

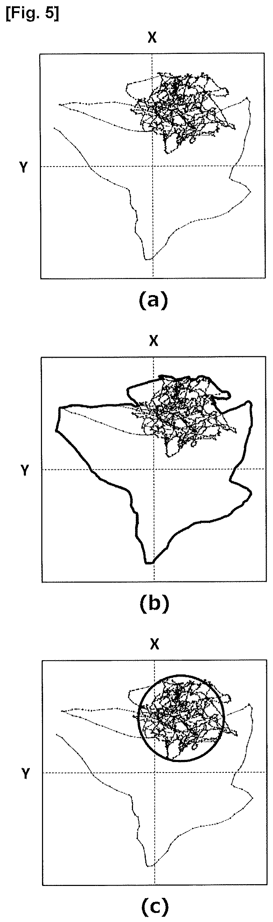

To diagnose dizziness and equilibrium disorders, center of gravity sway measurement using a stabilometer is performed in general. As illustrated in FIG. 5(a), the results of center of gravity sway measurement using a stabilometer display the trajectory of center of gravity sway as a line. Quantitative evaluation is typically performed using the length of the line drawn as the trajectory (total trajectory length) or the area enclosed by the perimeter set to surround the line (perimeter area, see FIG. 5(b)). However, previous evaluation methods have sometimes failed to properly evaluate the effectiveness of the trajectory, as they include trajectories unrelated to dizziness, such as those caused by temporary unsteadiness immediately after the start of measurement. In this regard, the inventors have adopted an evaluation method that introduces a new evaluation index that focuses on the aggregation density of the point cloud (plots) constituting the drawn trajectory.

As an example of a new evaluation index that focuses on the aggregation density of the point cloud (plots) constituting the drawn trajectory, the area of an approximate circle (90% approximate circle area) that represents 90% of the total number of plots, with the center of gravity of the plots constituting the drawn trajectory as its center, can be calculated and used (see FIG. 5(c)). The 90% approximate circle area can be calculated as follows:
(1) calculate the central coordinates (center of gravity of the trajectory) of a rectangle composed of the width (length between the minimum and maximum values on the x-axis coordinate) and height (length between the minimum and maximum values on the y-axis coordinate) of the trajectory of the center of gravity sway;
(2) calculate the distance from the center of gravity for each plot;
(3) sort the plots in order of shortest distance from the center of gravity;
(4) extract the plots corresponding to 90% of the total number of plots; and
(5) calculate the area of a circle centered on the coordinates of the center of gravity, with the radius from the extracted plot to the center of gravity.

The new evaluation index used for evaluation is not limited to the above-described 90% approximation circle area, provided that it is based on the aggregation density of the point cloud (plots) constituting the drawn trajectory. For example, it is also possible to extract plots to be evaluated using the variance or standard deviation of the distance from the center of gravity of the plots, and calculate the area of a circle defined based on those plots, or perform elliptical approximation (calculate the area of an ellipse).

### Experimental Method

In various experiments conducted to confirm the effectiveness of the present invention, the center of gravity sway measurement using a stabilometer was performed. The stabilometer used was one (specially designed, home-made one) with load sensors placed at the four corners of the examination table. The sampling frequency for the center of gravity sway was set to 60 hertz, and plot data of 3,600 points was acquired per minute. The scheme of experimental method employed was either a rotational load test (similar to that described in Patent Document 1), in which the subject was seated in a rotating chair and stimulated while the chair was rotating, or a rubber load test, a type of the center of gravity sway measurement performed clinically. In the rotational load test, a rotating plate (specially designed, home-made one) rotating at a constant speed (12 rpm) was used as the rotation mechanism for applying the rotational load to the subject. In the rubber load test, foam rubber (available from 5 Billion Fitness) was set on the examination table of the stabilometer and used.

Body equilibrium during the standing posture is maintained by processing inputs from the sense of equilibrium (vestibular sensation), sense of vision, and somatosensory sense in the central nervous system and outputting them to the skeletal muscles of the limbs/trunk. Evaluation of the body equilibrium during the standing posture is performed in a state of applying load to one or more of the input systems: inputs of the sense of equilibrium (vestibular sensation), sense of vision, and somatosensory sense. The present inventors employed the above-described rotational load test scheme and rubber load test scheme to evaluate the dizziness reduction effect due to a stimulus.

The effects of vestibular function stimulation were evaluated after providing sufficient explanation to the subjects and obtaining their consent. The two test schemes described below were conducted, and the selection was made with consideration for the recipients' age, preferences, etc.

The rotational load test scheme induces temporary dizziness due to the rotational load, and the recipient is therefore likely to actually feel the effects of the dizziness treatment stimulus. It is to be noted, however, that the temporary dizziness caused by the rotational load gradually subsides over time. Because the sensation of temporary dizziness and the time it takes for it to subside are easily affected by the recipient's physical condition and diurnal fluctuations, the effects of the recipient's physical condition and diurnal fluctuations are eliminated by dividing the results of the center of gravity sway measurement before and after the rotational load. To this end, the test has to be conducted twice, with and without the stimulus, and a sufficient interval has to be provided until the next measurement to eliminate the possibility that the temporary dizziness caused by the rotational load will affect the next measurement, making the evaluation time-consuming. Furthermore, the rotational load can be quite burdensome for some recipients, and even healthy individuals with impaired vestibular function due to aging or other factors may experience nausea symptoms (such as headaches and nausea) after the rotational load.

The rubber load test scheme allows simple and rapid evaluation to be carried out. The rubber load test scheme does not require the large-scale equipment required for applying a rotational load to the recipient, as is the case with the rotational load test scheme. Furthermore, because the load is applied only while the recipient is standing on the rubber during measurement, the burden on the recipient is extremely small.

Experiment of the rotational load test scheme was conducted using the following procedure. First, the subject (recipient) was asked to stand on the stabilometer, the center of gravity sway measurement was performed for one minute while standing with the eyes and legs closed (Procedure A1), and the 90% approximation circle area was calculated for the results of the center of gravity sway measurement before the rotational load obtained in Procedure A1 (Procedure A2). The subject was then given a one-minute rest (Procedure A3). Subsequently, the subject was asked to stand on the rotation mechanism in a chair and a rotational load was applied for one minute (Procedure A4). The subject was asked to stand on the stabilometer again and the center of gravity sway measurement was performed for one minute while standing with the eyes and legs closed (Procedure A5), and the 90% approximation circle area was calculated for the results of the center of gravity sway measurement before the rotational load obtained in Procedure A5 (Procedure A6). After an interval of at least 30 minutes, Procedures A1 to A6 are carried out again (Procedures B1 to B6). In Procedure B3, which corresponds to Procedure A3, the one-minute rest period is not provided, and instead, a stimulus is applied to the subject for one minute using the vestibular function improvement device. Therefore, Procedure A6 obtains the 90% approximate circle area after the rotational load without the stimulus, while Procedure B6 obtains the 90% approximate circle area after the rotational load with the stimulus. X1 is defined as a value obtained by dividing the 90% approximate circle area obtained in Procedure A6 by the 90% approximate circle area obtained in Procedure A2. Likewise, X2 is defined as a value obtained by dividing the 90% approximate circle area obtained in Procedure B6 by the 90% approximate circle area obtained in Procedure B2. Furthermore, the value obtained by dividing X2 by X1 indicates the degree of dizziness, which will be referred to below as a sway area ratio. A sway area ratio less than 1 indicates the dizziness reduction effect, while a ratio greater than 1 indicates a worsening of dizziness.

On the other hand, experiment of the rubber load test scheme was conducted using the following procedure. First, the subject (recipient) was asked to stand on the stabilometer on which the foam rubber was set, the center of gravity sway measurement was performed for one minute while standing with the eyes and legs closed (Procedure C1), and the 90% approximation circle area was calculated for the results of the center of gravity sway measurement before the rotational load obtained in Procedure C1 (Procedure C2). The subject was then given a stimulus for one minute using the vestibular function improvement device (Procedure C3). Subsequently, the subject was asked again to stand on the stabilometer on which the foam rubber was set, the center of gravity sway measurement was performed for one minute while standing with the eyes and legs closed (Procedure C4), and the 90% approximation circle area was calculated for the results of the center of gravity sway measurement before the rotational load obtained in Procedure C4 (Procedure C5). Y1 is defined as a value obtained by dividing the 90% approximate circle area obtained in Procedure C5 by the 90% approximate circle area obtained in Procedure C2. Y1 indicates the degree of dizziness, which will be referred to below as a sway area ratio. The evaluation of the sway area ratio is the same as for the rotational load test scheme.

Thus, the same indicator, sway area ratio, is used for both the rotational load test scheme and the rubber load test scheme, allowing for equivalent evaluation of treatment effectiveness using both the test schemes.

### Experiment 1

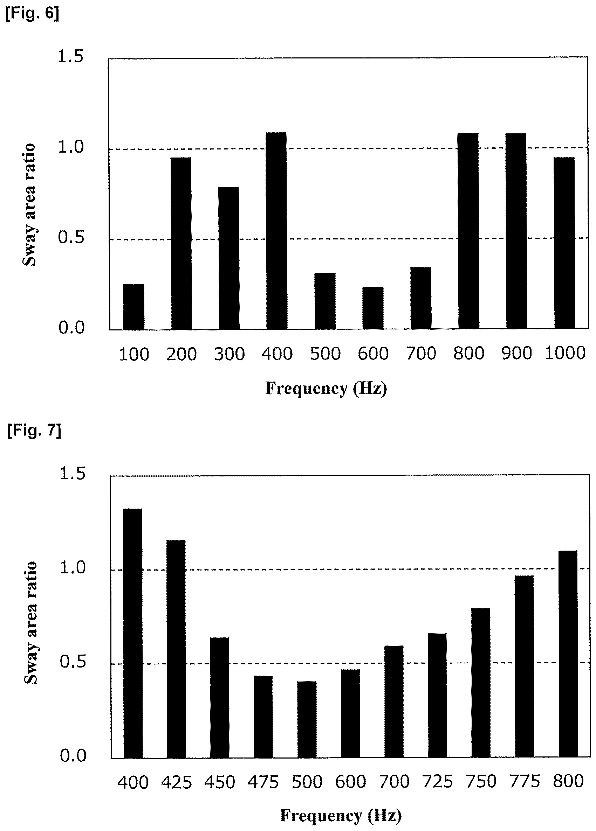

Following the above-described experimental procedure (rotational load test scheme), the vestibular function improvement device was used to generate a signal causing vibrations corresponding to pure tones with frequency components in 100-hertz increments in a range of 100 to 1000 hertz. A sound stimulus based on that signal was given to a subject (healthy individual, n=1) via headphones (available from Audio-Technica, model number: ATH-WS990BT). The results are listed in Table 1. FIG. 6 also illustrates the results of Table 1 presented as a graph, with the frequency on the horizontal axis and the sway area ratio on the vertical axis.

**[Table 1]**

| | | Frequency [Hz] | Sway area ratio |
|---|---|---|---|
| | Experiment 1-0 | - | 1.00 |
| | Experiment 1-1 | 100 | 0.26 |
| | Experiment 1-2 | 200 | 0.95 |
| | Experiment 1-3 | 300 | 0.79 |
| | Experiment 1-4 | 400 | 1.09 |
| Subject 1 | Experiment 1-5 | 500 | 0.31 |
| | Experiment 1-6 | 600 | 0.23 |
| | Experiment 1-7 | 700 | 0.34 |
| | Experiment 1-8 | 800 | 1.08 |
| | Experiment 1-9 | 900 | 1.08 |
| | Experiment 1-10 | 1,000 | 0.95 |

As listed in Table 1 and illustrated in FIG. 6, in addition to a 100 hertz pure tone, which is known to exhibit the dizziness reduction effect as a publicly known technique, pure tones in a range of 500 to 700 hertz were also confirmed to be effective.

### Experiment 2

Following the above-described experimental procedure (rubber load test scheme), the vestibular function improvement device was used to generate a signal causing vibrations corresponding to pure tones with frequency components in a range of 400 to 800 hertz, in 25-hertz increments for 500 hertz or lower and 700 hertz or higher and in 100-hertz increments in a range of 500 to 700 hertz. A sound stimulus based on that signal was given to a subject (healthy individual, n=1) via headphones (same as above). The results are listed in Table 2. FIG. 7 also illustrates the results of Table 2 presented as a graph, with the frequency on the horizontal axis and the sway area ratio on the vertical axis.

**[Table 2]**

| | | Frequency [Hz] | Sway area ratio |
|---|---|---|---|
| | Experiment 2-1 | 400 | 1.33 |
| | Experiment 2-2 | 425 | 1.16 |
| | Experiment 2-3 | 450 | 0.64 |
| | Experiment 2-4 | 475 | 0.44 |
| | Experiment 2-5 | 500 | 0.41 |
| Subject 1 | Experiment 2-6 | 600 | 0.47 |
| | Experiment 2-7 | 700 | 0.59 |
| | Experiment 2-8 | 725 | 0.66 |
| | Experiment 2-9 | 750 | 0.79 |
| | Experiment 2-10 | 775 | 0.96 |
| | Experiment 2-11 | 800 | 1.10 |

As listed in Table 2 and illustrated in FIG. 7, apparent effects were observed with pure tones in a range of 450 to 750 hertz, and slight improvement was also confirmed with a pure tone of 775 hertz.

### Experiment 3

Since Experiments 1 and 2 were conducted for an individual as the subject, it can be said that the results do not reflect individual differences. Previous research on vestibular stimuli using VEMP (Vestibular Evoked Myogenic Potential) test has reported that, for example, in patients with Meniere's disease, the frequency (characteristic frequency) highly responsive to vestibular stimuli shifts toward higher frequencies. It has also been reported that the otolithic layer, which is involved in vestibular stimuli, exhibits morphological changes, such as enlargement of the otoliths, with age. On the basis of these reports, the following experiment was conducted on five subjects of different ages and with or without ear disease to confirm whether the characteristic frequencies confirmed in Experiments 1 and 2 varied among individuals.

Following the above-described experimental procedure (rubber load test scheme), the vestibular function improvement device was used to generate a signal causing vibrations corresponding to pure tones with frequency components in 500-hertz increments in a range of 500 to 2000 hertz. A sound stimulus based on that signal was given to the subjects via headphones (same as above). The results are listed in Table 3. Subject 5 has ear disease (patulous Eustachian tube, viral hearing loss).

**[Table 3]**

| | | Frequency [Hz] | Sway area ratio |
|---|---|---|---|
| Subject 1 29 y/o Male | Experiment 3-1 | 500 | 0.70 |
| | Experiment 3-2 | 750 | 0.83 |
| | Experiment 3-3 | 1,000 | 1.21 |
| | Experiment 3-4 | 1,500 | 1.54 |
| | Experiment 3-5 | 2,000 | 1.74 |
| Subject 2 29 y/o Female | Experiment 3-6 | 500 | 0.85 |
| | Experiment 3-7 | 750 | 0.59 |
| | Experiment 3-8 | 1,000 | 0.94 |
| | Experiment 3-9 | 1,500 | 0.99 |
| | Experiment 3-10 | 2,000 | 1.10 |
| Subject 3 46 y/o Male | Experiment 3-11 | 500 | 0.87 |
| | Experiment 3-12 | 750 | 0.93 |
| | Experiment 3-13 | 1,000 | 0.81 |
| | Experiment 3-14 | 1,500 | 0.60 |
| | Experiment 3-15 | 2,000 | 0.73 |
| Subject 4 56 y/o Male | Experiment 3-16 | 500 | 1.16 |
| | Experiment 3-17 | 750 | 0.85 |
| | Experiment 3-18 | 1,000 | 0.64 |
| | Experiment 3-19 | 1,500 | 1.02 |
| | Experiment 3-20 | 2,000 | 0.99 |
| Subject 5 58 y/o Female | Experiment 3-21 | 500 | 0.81 |
| | Experiment 3-22 | 750 | 0.76 |
| | Experiment 3-23 | 1,000 | 0.68 |
| | Experiment 3-24 | 1,500 | 0.87 |
| | Experiment 3-25 | 2,000 | 1.23 |

As listed in Table 3, the frequency range in which the vibration area ratio fell below 1, i.e., the therapeutic effect, varied among individuals. Furthermore, the frequency with the greatest therapeutic effect in that range (highlighted in bold in Table 3) also varied among individuals, with a clear shift toward higher frequencies primarily due to age. High-frequency bands are thought to be particularly effective for vertigo patients. It is therefore believed that it would be more effective to adjust the frequency range and sound pressure level of the therapeutic stimulus appropriately in accordance with the recipient's condition.

Thus, the frequency range with the most therapeutic effect varies among individuals, with this range being 450 to 1500 hertz. It has been confirmed that it is important for the therapeutic stimulus to include frequency components in this range.

### Experiment 4

In conducting experiments in which subjects were given stimuli containing various frequency components, the inventors have conceived of the possibility that high-frequency components attenuate the effects of vestibular function improvement. In other words, high-frequency components have a negative impact on the vestibular function improvement effect. To confirm this, following the above-described experimental procedure, the vestibular function improvement device was used to generate a stimulus sound composed of a pure tone with a 100 hertz frequency component, which is known to have a vestibular function improvement effect, and a pure tone with a 600 hertz frequency component, which is also known to have a vestibular function improvement effect. This stimulus sound was then further synthesized with a pure tone with a component in a high-frequency band (non-effective frequency) to generate a signal causing the corresponding vibration. The sound stimulus based on this signal was then given to subjects (healthy individuals, n=2) via headphones (same as above). Multiple experiments were conducted while varying the frequency component in the high-frequency band from 2000 hertz to 8000 hertz. The rotational load test scheme was used for Subject 1, and the rubber load test scheme was used for Subject 4. The results are listed in Table 4.

**[Table 4]**

| | | Effective frequency [Hz] | Non-effective frequency [Hz] | Sway area ratio |
|---|---|---|---|---|
| | Experiment 4-0 | - | - | 1.00 |
| | Experiment 4-1 | | - | 0.16 |
| Subject 1 | Experiment 4-2 | 100 + 600 | 2,000 | 0.28 |
| | Experiment 4-3 | | 4,000 | 0.34 |
| | Experiment 4-4 | | 5,000 | 0.49 |
| | Experiment 4-5 | | 6,000 | 0.57 |
| | Experiment 4-6 | | 8,000 | 1.32 |
| Subject 4 | Experiment 4-7 | 100 + 600 | - | 0.86 |
| | Experiment 4-8 | | 4,000 | 0.95 |
| | Experiment 4-9 | | 8,000 | 1.73 |

As listed in Table 4, for Subject 1, the sway area ratio was 0.16 when the pure tone with non-effective frequency was not synthesized, indicating that the stimulus sound synthesized from a 100 hertz pure tone and a 600 hertz pure tone had a significant effect on improving the vestibular function. However, when pure tones with non-effective frequencies in a high-frequency band of 2000 hertz or higher were further synthesized, the sway area ratio increased with increasing frequency, indicating a diminished effect on improving the vestibular function. In particular, when a pure tone with a non-effective frequency of 8000 hertz was synthesized, it has been found that unsteadiness due to the rotational load worsened compared to before the stimulus sound synthesized from a 100 hertz pure tone and a 600 hertz pure tone is applied.

Also for Subject 4, the sway area ratio was 0.86 when no pure tones with non-effective frequencies were synthesized. However, when pure tones with non-effective frequencies in a high-frequency band of 4000 hertz or higher were further synthesized, the sway area ratio increased, indicating a diminished effect on improving the vestibular function. In particular, when a pure tone with a non-effective frequency of 8000 hertz was synthesized, not only was the vestibular function improvement effect lost, but the dizziness state worsened.

It has thus been confirmed from the results of Experiment 4 that even when the subject is given a sound stimulus containing effective frequency components that have a vestibular function improvement effect, a sound stimulus containing frequency components in a high-frequency band of 2000 hertz or higher diminishes the vestibular function improvement effect.

Up to this point, evaluation has been performed on the vestibular function improvement effect (dizziness reduction effect) of applying a sound stimulus using headphones, but a similar evaluation has been carried out on the vestibular function improvement effect of applying a vibration stimulus using bone conduction earphones, confirming that the frequency range exhibiting the vestibular function improvement effect is the same.

### Experiment 5

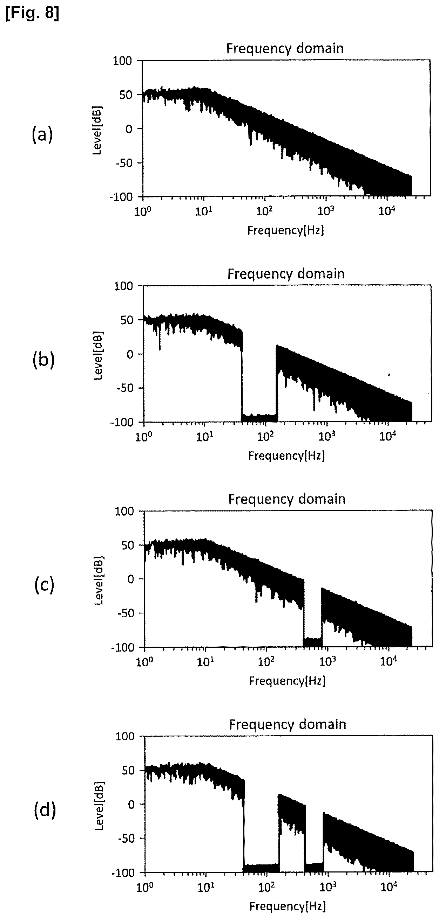

Following the above-described experimental procedure (rotational load test scheme), the vestibular function improvement device was used to create a Brownian noise sound source and noise sources with a partial attenuated frequency band of Brownian noise as illustrated in FIG. 8. Signals were generated to cause vibrations corresponding to the noises, and sound stimuli based on these signals were given to a subject (healthy individual, n=1) via headphones (available from Audio-Technica, model number: ATH-WS990BT). FIG. 8 is a set of semi-logarithmic graph with the vertical axis representing sound pressure level and the horizontal axis representing frequency, in which (a) represents Brownian noise, (b) represents Brownian noise with the 40 to 150 hertz frequency band cut off, (c) represents Brownian noise with the 400 to 800 hertz frequency band cut off, and (d) represents Brownian noise with the 40 to 150 hertz and 400 to 800 hertz frequency bands cut off. The experimental results are listed in Table 5.

**[Table 5]**

| | | Noise | Band-cut processing | Sway area ratio |
|---|---|---|---|---|
| | Experiment 5-0 | - | - | 1.00 |
| | Experiment 5-1 | Brownian noise | - | 0.33 |
| Subject 1 | Experiment 5-2 | | 40-150 Hz | 0.23 |
| | Experiment 5-3 | | 400-800 Hz | 0.25 |
| | Experiment 5-4 | | 40-150 Hz | 1.85 |
| | | | 400-800 Hz | |

As listed in Table 5, the vestibular function improvement effect was confirmed even when the subject was given a sound stimulus of Brownian noise, which is a noise containing specific frequency components at least in a range of 450 to 1500 hertz. It can be seen that band-cut processing is effective whether it cuts the 40 to 150 hertz frequency band of Brownian noise or the 400 to 800 hertz frequency band of Brownian noise, but cutting both the 40 to 150 hertz and 400 to 800 hertz frequency bands of Brownian noise eliminates the vestibular function improvement effect.

### Experiment 6

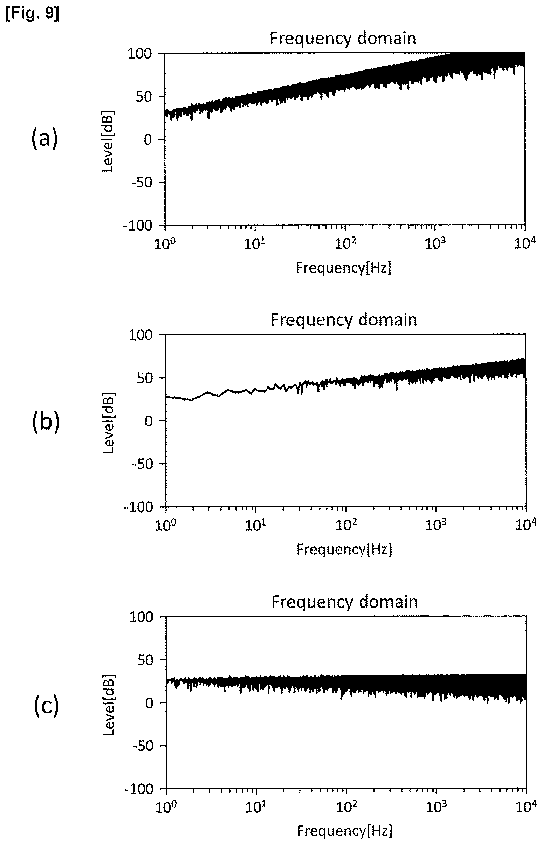

Following the above-described experimental procedure (rotational load test scheme), the vestibular function improvement device was used to generate signals causing vibrations corresponding to the purple noise, blue noise, and white noise illustrated in FIG. 9. Sound stimuli based on these signals were given to a subject (healthy individual, n=1) via headphones (available from Audio-Technica, model number: ATH-WS990BT). FIG. 9 is a set of semi-logarithmic graph with the vertical axis representing sound pressure level and the horizontal axis representing frequency, in which (a) represents purple noise, (b) blue noise, and (c) white noise. The experimental results are listed in Table 6.

**[Table 6]**

| | | Noise | Sway area ratio |
|---|---|---|---|
| | Experiment 6-0 | - | 1.00 |
| Subject 1 | Experiment 6-1 | Purple noise | 1.29 |
| | Experiment 6-2 | Blue noise | 1.30 |
| | Experiment 6-3 | White noise | 1.35 |

As listed in Table 6, when the subject was given the sound stimulus of purple, blue, or white noise with a high sound pressure level in a frequency band of 2000 hertz or higher, even noise containing specific frequency components in a range of 450 to 1500 hertz, no vestibular function improvement effect was confirmed. It has been confirmed from this that when giving the subject a sound stimulus of noise, it is preferred to give noise in which the sound pressure level of a frequency band of 2000 hertz or higher is lower than the sound pressure level of a frequency band in a range of 450 to 1500 hertz.

### Experiment 7

In Experiment 5, signals were generated to cause vibrations corresponding to the Brownian noise and noise with a partial band-cut processed frequency band of Brownian noise as illustrated in FIG. 8, and sound stimuli based on these signals were given to the subject. To give the noises illustrated in FIG. 8 as sound stimuli, bone conduction earphones (available from Aftershokz, Aeropex) were connected to the vestibular function improvement device instead of headphones, and the vibration stimuli were given to a subject (healthy individual, n=1). The experimental results are listed in Table 7.

**[Table 7]**

| | | Noise | Band-cut processing | Sway area ratio |
|---|---|---|---|---|
| | Experiment 7-0 | - | - | 1.00 |
| | Experiment 7-1 | Brownian noise | - | 0.15 |
| Subject 1 | Experiment 7-2 | | 40-150 Hz | 0.22 |
| | Experiment 7-3 | | 400-800 Hz | 0.16 |
| | Experiment 7-4 | | 40-150 Hz | 1.30 |
| | | | 400-800 Hz | |

As listed in Table 7, also when the vibration stimuli were given instead of sound stimuli, experimental results similar to those obtained with sound stimuli were obtained. In other words, the vestibular function improvement effect was confirmed even when the subject was given a sound stimulus of Brownian noise, which is a noise containing specific frequency components at least in a range of 450 to 1500 hertz. It can be seen that band-cut processing is effective whether it cuts the 40 to 150 hertz frequency band of Brownian noise or the 400 to 800 hertz frequency band of Brownian noise, but cutting both the 40 to 150 hertz and 400 to 800 hertz frequency bands of Brownian noise eliminates the vestibular function improvement effect.

### Experiment 8

In Experiment 6, signals were generated to cause vibrations corresponding to the purple, blue, and white noises illustrated in FIG. 9, and sound stimuli based on these signals were given to the subject, but the effects of giving these signals as vibration stimuli are confirmed. As in Experiment 7, instead of headphones, bone conduction earphones (available from Aftershokz, Aeropex) were connected to the vestibular function improvement device, and vibration stimuli based on the noises illustrated in FIG. 9 were given to a subject (healthy individual, n=1). The experiment results are listed in Table 8.

**[Table 8]**

| | | Noise | Sway area ratio |
|---|---|---|---|
| | Experiment 8-0 | - | 1.00 |
| Subject 1 | Experiment 8-1 | Purple noise | 1.95 |
| | Experiment 8-2 | Blue noise | 0.44 |
| | Experiment 8-3 | White noise | 0.26 |

As listed in Table 8, when the subject was given the vibration stimulus of purple noise with a high sound pressure level in a frequency band of 2000 hertz or higher, even noise containing specific frequency components in a range of 450 to 1500 hertz, no vestibular function improvement effect was observed, as was the case with sound stimulus. On the other hand, when white noise or blue noise was given to the subject as a sound stimulus, there was no vestibular function improvement effect (Experiment 6-2), but when given as a vibration stimulus, the vestibular function improvement effect was confirmed. In other words, when a vibration stimulus with power spectral density proportional to the frequency range from the first power (blue noise) to the second power (purple noise) was given, it was found to be effective in improving the vestibular function. This is thought to be because the bone conduction propagation path is complex, and high-frequency bands are attenuated by skin, bone tissue, etc. in the process of the vibrations reaching the inner ear, resulting in that the white noise is transmitted to the inner ear as noise with frequency characteristics similar to those of Brownian noise.

### Experiment 9

In Experiment 7, a vibration stimulus based on Brownian noise illustrated in FIG. 8(a) was given to the anterior part of the ear using the bone conduction earphones, but the effects of applying vibrations to other sites (mandible, nasal bone, mastoid process, and parietal area) by contact are confirmed. The bone conduction earphones used in Experiment 7 were connected to the vestibular function improvement device, and the vibration stimulus based on the noise illustrated in FIG. 8(a) was given to a subject (healthy individual, n=1). The experimental results are listed in Table 9.

**[Table 9]**

| | | Noise | Vibration applied site | Sway area ratio |
|---|---|---|---|---|
| | Experiment 9-1 | | Ear anterior part | 0.27 |
| | Experiment 9-2 | | Mandible | 0.24 |
| | Experiment 9-3 | | Nasal bone | 0.37 |
| Subject 1 | Experiment 9-4 | Brownian noise | mastoid process | 0.13 |
| | Experiment 9-5 | | parietal area | 0.32 |

As listed in Table 9, also when the vibration was applied to the mandible, nasal bone, mastoid process, or parietal area, the same effect was observed as when the vibration stimulus was applied to the anterior part of the ear using bone conduction earphones.

### Experiment 10

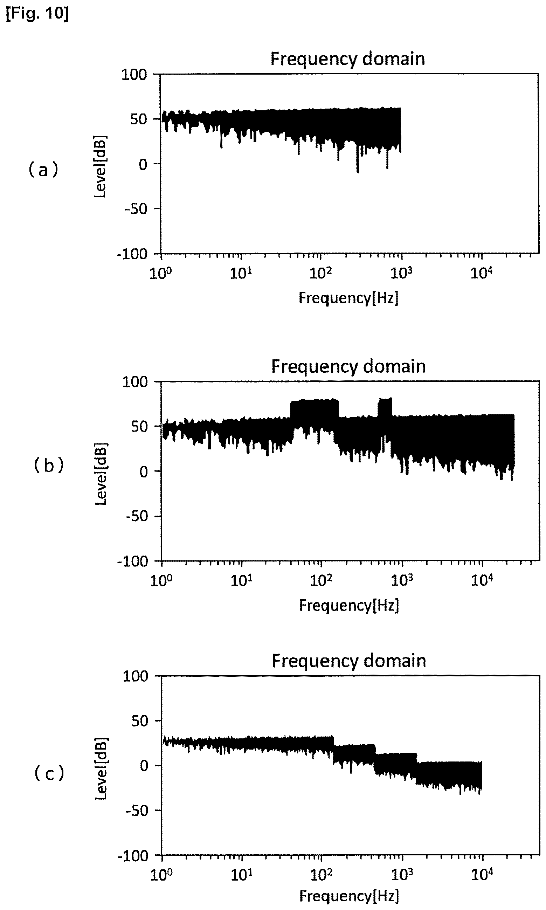

Following the above-described experimental procedure (rotational load test scheme), signals were generated to cause vibrations corresponding to the processed white noises illustrated in FIG. 10 using the vestibular function improvement device, and sound stimuli based on these signals were given to a subject (healthy individual, n=1) via headphones (available from Audio-Technica, model number: ATH-WS990BT). FIG. 10 is a set of semi-logarithmic graphs with the vertical axis representing sound pressure level and the horizontal axis representing frequency, in which (a) represents white noise with a frequency band of 2000 hertz or higher cut using band-cut processing, (b) represents white noise with the sound pressure level enhanced in both the 50 to 140 hertz and 500 to 700 hertz frequency bands, and (c) represents white noise with attenuation processing to decrease the sound pressure level in frequency bands of 150 to 450 hertz, 450 to 1500 hertz, and 1500 hertz or higher in this order. The experiment results are listed in Table 10.

**[Table 10]**

| | | Processing content | Sway area ratio |
|---|---|---|---|
| | Experiment 10-0 | - | 1.00 |
| | Experiment 10-1 | Cut high-frequency band | 0.26 |
| Subject 1 | Experiment 10-2 | Emphasize effective frequency band | 0.12 |
| | Experiment 10-3 | Attenuate high-frequency band stepwise | 0.24 |

As listed in Table 10, even when white noise was given to the subject as a sound stimulus, the vestibular function improvement effect was confirmed by cut-processing a frequency band of 2000 hertz or higher, emphasis-processing the effective frequency bands (50 to 140 hertz and 500 to 700 hertz), and attenuation-processing the high-frequency band in a stepped manner.

### Experiment 11

Following the above-described experimental procedure (rotational load test scheme), the vestibular function improvement device was used to synthesize the Brownian noise illustrated in FIG. 8(a) with classical music (Chopin, Nocturne Op. 9-2), and the sound stimulus thus obtained was given to a subject (healthy individual, n=1) via headphones (available from Audio-Technica, model number: ATH-WS990BT). For comparison, an experiment was also conducted in which a sound stimulus based on classical music without Brownian noise was given. The experimental results are listed in Table 11.

**[Table 11]**

| | | Noise | Noise | way area ratio |
|---|---|---|---|---|
| | Experiment 11-0 | - | - | 1.00 |
| Subject 1 | Experiment 11-1 | Chopin, Nocturne | Brownian noise | 0.12 |
| | Experiment 11-2 | | - | 0.86 |

As listed in Table 11, sufficient dizziness reduction effect was not confirmed by providing the subject with a music-only sound stimulus, but sufficient dizziness reduction effect was recognized by providing the subject with a music sound stimulus mized with Brownian noise.

### Experiment 12

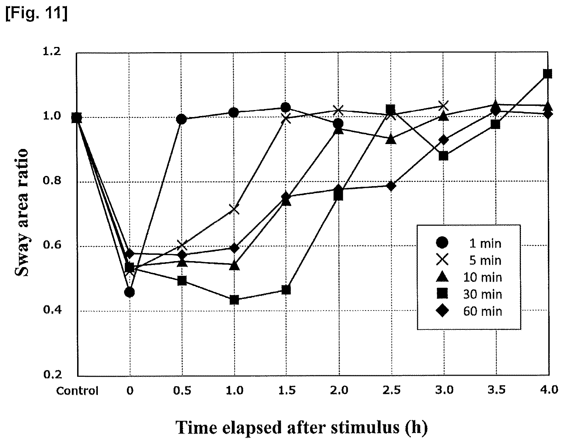

Following the above-described experimental procedure (rubber load test scheme), the vestibular function reduction device was used to generate a signal causing vibrations corresponding to the Brownian noise illustrated in FIG. 8(a), and a sound stimulus based on this signal was given to a subject (healthy individual, n=1) via headphones (available from Audio-Technica, model number: ATH-WS990BT). Here, in Procedure B2, the duration of the stimulus given to the subject using the vestibular function improvement device was varied from 1 minute, 5 minutes, 10 minutes, 30 minutes, and 60 minutes to verify the effect of varying the stimulus duration. In addition, the center of gravity sway measurement of Procedure B3 was conducted every 30 minutes starting immediately after stimulus application to verify the duration of the stimulus's effect. FIG. 11 illustrates the results of the verification, represented as a graph with the measurement timing on the horizontal axis and the sway area ratio on the vertical axis. The measurement was performed three times, and the average values are illustrated in FIG. 11.

As illustrated in FIG. 11, a tendency has been confirmed that the longer the stimulus duration, the longer the duration of the effect. With a stimulus duration of 1 minute, measurements taken 30 minutes after the stimulus were already equivalent to measurements taken without stimulus, but with a stimulus duration of 5 minutes or longer, the effect persisted even one hour after the stimulus. With a stimulus duration of 30 or 60 minutes, the effect was confirmed to persist even more than two hours after the stimulus. However, a stimulus duration of 10 minutes or longer is not expected to significantly increase the duration of the effect, and given the significant physical and time burden on the recipient, it is believed that a longer stimulus duration is not necessarily a better outcome.

### Experiment 13

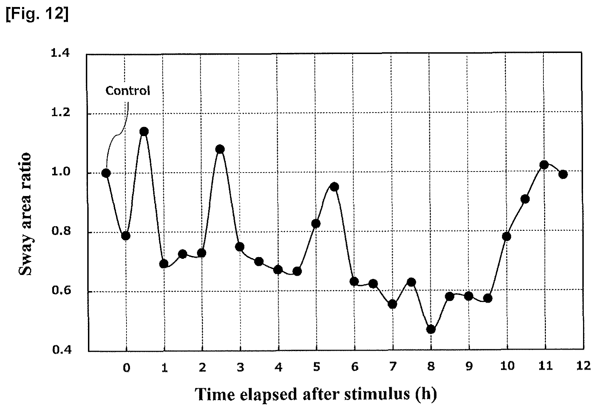

Following the above-described experimental procedure (rubber load test scheme), the vestibular function reduction device was used to generate a signal causing vibrations corresponding to the Brownian noise illustrated in FIG. 8(a), and a sound stimulus based on this signal was given to a subject (healthy individual, n=1) via headphones (available from Audio-Technica, model number: ATH-WS990BT). Here, the center of gravity sway measurement of Procedure B3 was conducted every 30 minutes, starting immediately after stimulus application, and if the measurement results were equivalent to those without stimulus application, another one-minute stimulus was applied before the next measurement 30 minutes later. Such an experiment was performed to verify the duration of the effect when the stimulus was repeated. FIG. 12 illustrates the results of the verification, represented as a graph with the measurement timing on the horizontal axis and the 90% approximate circular area on the vertical axis. The measurement was performed three times, and the average values are illustrated in FIG. 11.

As confirmed in Experiment 13, when the stimulus duration was 1 minute, measurements taken 30 minutes after the stimulus were already equivalent to measurements taken without any stimulus, but when a one-minute stimulus was applied again 30 minutes later (one hour after the first stimulus), the effect was observed in the measurement immediately thereafter. In addition, a tendency for the effect to last longer was recognized. This tendency was observed to become stronger with each additional one-minute stimulus. In other words, it has been confirmed that the duration of the effect is extended by applying multiple one-minute stimuli. Thus, while the effect lasted only a short time with a single stimulus, the duration was significantly extended with multiple stimuli. This carryover effect allows stimulation several times a day to maintain the effect for a long period of time, which is highly desirable from the perspective of reducing the burden on the recipient.

The various experiments were conducted above to confirm the effects of the present invention, and findings related to known techniques obtained from these experiments will be described. It has been known that applying sound of a specific frequency (50 to 140 hertz) to a subject at a specific volume level (70 to 85 decibels) exhibits an effect of reducing dizziness (Patent Document 1). Fortunately, however, from the results of Experiments 5, 7, 8, 9, 10, etc., it has been confirmed that the effect of reducing dizziness can be obtained with stimuli containing frequency components of 50 to 140 hertz even at a volume level of 70 decibels or less.

For example, in Experiment 5, applying the Brownian noise illustrated in FIG. 8(a) to the subject leads to an effect of reducing dizziness, but applying the Brownian noise illustrated in FIG. 8(c) with the 400 to 800 hertz frequency band cut also reduces dizziness. On the other hand, the effect of reducing dizziness was not able to be confirmed for the Brownian noise illustrated in FIG. 8(d) with the 40 to 150 hertz and 400 to 800 hertz frequency bands cut. From this, it can be understood that the reason the Brownian noise illustrated in FIG. 8(c) with the 400 to 800 hertz frequency band cut leads to an effect of reducing dizziness is because the noise contains a frequency band of 50 to 140 hertz.

When the processed Brownian noise illustrated in FIG. 8(c) was played in the present experiment, the sound pressure level for each frequency (50 to 140 hertz) was measured and found to be less than 70 decibels. This suggests that a stimulus containing frequency components of 50 to 140 hertz can obtain an effect of reducing dizziness even at a sound pressure level below 70 decibels.

A stimulus containing frequency components of 50 to 140 hertz does not necessarily have to be noise, and it may be a pure tone with a specific frequency component in the 50 to 140 hertz range, or a combination of a plurality of such pure tones. By giving a subject a stimulus containing frequency components in the 50 to 140 hertz range, dizziness of a recipient can be reduced even when the sound pressure level of that stimulus is below 70 decibels.

### Description of Reference Signs

- 100, 200: Vestibular function improvement device
- 1: Device main body
- 2: Headphone
- 3: Bone conduction earphones

## Claims

1. A vestibular function improvement device comprising:
a generation unit that generates an electric signal that causes a vibration including a frequency component in a range of 450 to 1500 hertz; and
an output unit that outputs a stimulus based on the electric signal.

2. The vestibular function improvement device according to claim 1,
wherein the stimulus output by the output unit is transmitted to a vestibular system of an inner ear.

3. The vestibular function improvement device according to claim 1,
wherein the stimulus is a sound stimulus.

4. The vestibular function improvement device according to claim 1,
wherein the stimulus is a vibration stimulus.

5. The vestibular function improvement device according to claim 1,
wherein the output unit outputs the stimulus as noise including frequency components across a predetermined range.

6. The vestibular function improvement device according to claim 5,
wherein a sound pressure level of a high-frequency band in the noise is lower than the sound pressure level of a frequency band in a range of 450 to 1500 hertz.

7. The vestibular function improvement device according to claim 6,
wherein the high-frequency band is a frequency band of 2000 hertz or higher.

8. The vestibular function improvement device according to claim 5,
wherein the output unit outputs the stimulus in a form of Brownian noise or pink noise.

9. The vestibular function improvement device according to claim 1,
wherein the stimulus is a vibration stimulus, and
the output unit outputs the stimulus in a form of white noise.

10. The vestibular function improvement device of any one of claims 1 to 9, wherein the output unit outputs the stimulus after mixing it with a sound different from the stimulus.

11. A vestibular function improvement method comprising:
generating an electric signal that causes a vibration including a frequency component in a range of 450 to 1500 hertz;
outputting a stimulus based on the electric signal; and
transmitting the stimulus to a vestibular system of an inner ear.
